# FASCICULE DE BREVET EUROPEEN

(11) **EP 1 325 762 B1**
(45) Date de publication et mention de la délivrance du brevet: **31.05.2006**
(21) Numéro de dépôt: 03290020.1
(22) Date de dépôt: 06.01.2003
(51) Int. Cl.: A61M 16/20

(54) **Valve à la demande utilisable en oxygénothérapie**
Bedarfgesteuertes Ventil für die Oxygenotherapie
Demand valve for use in oxygenotherapy

(30) Priorité: 08.01.2002 FR 0200150
(43) Date de publication de la demande: 09.07.2003
(73) Titulaire: TAEMA, F-92182 Antony Cédex (FR)
(72) Inventeur: Meneuvrier, Matthieu, 91320 Wissous (FR); Bleys, Christian, 77000 Livry sur Seine (FR); Pingret, Stéphane, 91130 Ris-Orangis (FR)
(74) Mandataire: Pittis, Olivier

(56) Documents cités:
- EP-A- 0 937 640
- WO-A-90/01965
- FR-A- 2 763 382
- FR-A- 2 813 799
- US-A- 2 328 214

## Description

La présente invention concerne une valve à la demande utilisable en oxygénothérapie de patient et, plus précisément, une telle valve équipé d'un système de connexion rapide sur la sortie de gaz d'un débitmètre à sélecteur de débit ou d'un système équivalent permettant de délivrer un débit de gaz déterminé, soit un débit continu ladite connexion rapide comportant au moins deux positions de connexion.

Certaines personnes souffrant d'insuffisances respiratoires nécessitent d'avoir un apport d'oxygène complémentaire de manière à augmenter la proportion d'oxygène présent dans l'air ou le gaz qu'ils inspirent au delà de la teneur en oxygène qui y est habituellement présente.

Pour ce faire, il est habituel d'envoyer aux voies aériennes supérieures de ces personnes de l'oxygène au moyen de systèmes d'alimentation en gaz, qui peuvent être de plusieurs modèles différents, tels des masques respiratoires ou des dispositifs de type lunettes respiratoires.

A ce titre, on peut citer par exemple les documents EP-A-099283, US-A-4,648,395, DE-A-4309923, EP-A-991460 et EP-A-941136.

Toutefois, l'envoi d'oxygène vers les voies respiratoires d'un patient peut se faire soit en continu mais cela engendre des pertes de gaz, soit de manière synchronisée avec les phases respiratoires du patient, c'est-à-dire uniquement pendant les phases inspiratoires.

Différents solutions ont déjà été proposées pour synchroniser l'envoi du gaz avec les phases inspiratoires du patient.

Certaines d'entre elles fonctionnent à l'aide de capteurs ou d'autres dispositifs électriques ou électroniques qui détectent l'activité respiratoire du patient au niveau du masque respiratoire ou par d'autres moyens. A titre d'exemple, on peut citer le document EP-A-998318 qui décrit un pilotage basé sur un suivi de la teneur du sang en oxygène ou le document WO-01-43806 qui décrit un dispositif d'envoi de NO gazeux à un patient qui est contrôlé par une unité à microprocesseur.

A l'inverse, d'autres solutions beaucoup plus simples fonctionnent sur un mode entièrement pneumatique. Dans ce cas, ce sont les variations de pression du gaz inspiré ou expiré par le patient qui sont utilisées pour déterminer les moments où doit s'effectuer l'envoi du gaz vers le patient.

Parmi les dispositifs pneumatiques existants, les plus couramment utilisés sont les valves à la demande, encore appelées valves "économiseuses", qui contrôlent l'alimentation en gaz du patient de sorte que le gaz ne soit envoyé que lorsque le patient en demande, c'est-à-dire lorsqu'il commence à inspirer, ce qui permet d'éviter de gaspiller du gaz, lorsque le patient n'en a pas besoin.

On peut citer, par ailleurs, les documents suivants :
- EP-A-937640 portant sur un régulateur de gaz utilisable en plongée comprenant, lui aussi, un by-pass sur sa ligne principale servant à faciliter la respiration du plongeur.
- WO-A-90/01965 portant sur un système à valve utilisable sur les appareils d'oxygénothérapie à envoi séquentiel de gaz, lors des phases respiratoires. Ce système comporte deux lignes de gaz, l'une servant à envoyer le gaz sous forme de « pulses », l'autre servant à convoyer le gaz en continu. Le contrôle de l'envoi du gaz vers l'une ou l'autre de ces lignes se faisant grâce à une valve manuelle mobile entre deux positions stables dans lesquelles les entrées des deux lignes de gaz sont alternativement obturées ou ouvertes.
- FR-A-2763382 portant sur un régulateur de gaz avec dispositif de sécurité comprenant une ligne de by-pass destiné à être utilisé dans des conditions sévères : très basses pressions, environnements très pollués (pollution nucléaire, bactériologique ou chimique)
- FR-A-2813799 portant sur une valve économiseuse dont l'originalité semble porter sur l'utilisation d'une membrane pour contrôler l'écoulement du gaz. Ce document précise que le dispositif ne comporte pas de passage de dérivation reliant le passage d'alimentation à la chambre de distribution. Le document FR-A-2813799 est considéré comme l'état de la technique le plus proche.
- US-A-2,328,214 portant sur un régulateur d'oxygène.

Toutefois, les valves proposées par ces documents ne sont pas idéales car aucune de ces valves ne résout le problème d'une délivrance en continu du gaz, en particulier en cas de panne du mécanisme interne, sans avoir à déconnecter le conduit de sortie de la valve pour le rebrancher directement sur le raccord de sortie de la source de gaz.

Dit autrement, le problème qui se pose est qu'en cas de panne ou de défaut de leur mécanisme interne, les valves économiseuses connues ne possèdent pas de moyen aisé pour délivrer le gaz en mode continu. En effet, actuellement, en cas de panne ou de défaut du système, il faut déconnecter le tuyau de sortie de la valve économiseuse pour le rebrancher directement en sortie de débitmètre ou de la source de débit.

Le but de l'invention est alors de résoudre ce problème en proposant une valve améliorée permettant d'obtenir une délivrance en continu du gaz, en particulier en cas de panne ou de défaut de son mécanisme interne, sans avoir à déconnecter le conduit de sortie de la valve pour le rebrancher directement sur le raccord de sortie de la source de gaz.

La solution proposée par l'invention concerne alors une valve de distribution de gaz selon la revendication 1.

Selon le cas, la valve de l'invention peut comprendre l'une ou plusieurs des caractéristiques techniques suivantes :
- un logement est aménagé sur le passage principal d'acheminement de gaz, une paroi dudit logement portant l'orifice d'entrée de gaz et au moins une partie des moyens de connexion étant situés dans ledit logement ou au niveau de la paroi dudit logement.
- la pièce-manchon porte des moyens d'étanchéité sur sa paroi périphérique externe, de préférence les moyens d'étanchéité sont deux joints toriques espacés l'un de l'autre.
- lorsque la pièce-manchon est dans sa deuxième position stable, les moyens d'étanchéité portés par la paroi périphérique externe de la pièce-manchon empêchent toute introduction de gaz dans l'entrée amont du passage secondaire d'acheminement de gaz se raccordant fluidiquement au passage principal d'acheminement de gaz en amont desdits moyens à valve.
- un mécanisme de maintien permet un maintien de la pièce-manchon dans sa première position stable et/ou dans sa deuxième position stable.
- un mécanisme de libération coopérant avec le mécanisme de maintien permet la libération de la pièce-manchon lorsqu'elle est dans la première position stable et/ou dans la deuxième position stable, de préférence le mécanisme de libération comprend un bouton à actionnement digital. Dans un mode de réalisation préféré, le mécanisme de libération comprend un bouton à actionnement digital et un ressort de rappel lui permettant de reprendre sa position lorsque celui-ci n'est pas actionné.
- un moyen élastique situé dans le passage principal d'acheminement de gaz exerce une force de poussée sur la pièce-manchon dans le sens tendant à l'éloigner de l'entrée amont du passage secondaire.

Selon un autre aspect, l'invention porte aussi sur un ensemble formé par un débitmètre comportant un raccord de sortie sur lequel est connecté de manière solidaire une valve selon l'invention.

L'invention va maintenant être mieux comprise à l'aide de l'explication détaillée ci-après donnée en références aux figures annexées.

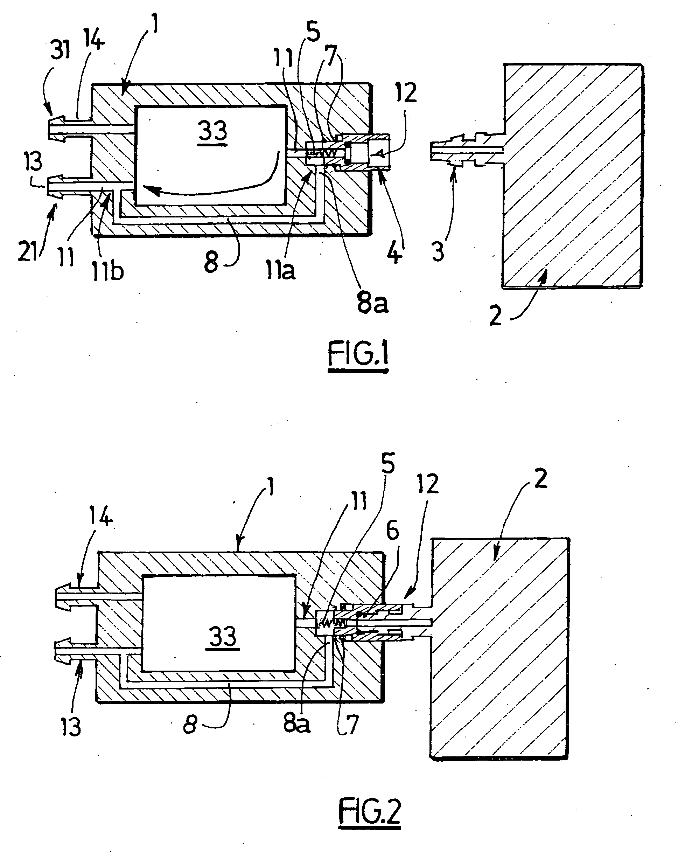

La figure 1 schématise, en vue en coupe, une valve économiseuse 1 selon l'invention qui est conçue pour se connecter sur le raccord ou l'olive 3 de sortie d'un débitmètre 2 monté sur la sortie d'une source de gaz sous pression, telle une bouteille d'oxygène ou une canalisation. La connexion de raccordement de la valve se fait essentiellement par l'intermédiaire d'une pièce-manchon mobile 4 ou fût mobile comportant un mécanisme permettant l'accrochage sur l'olive 3 du débitmètre. Un ressort 5 ou analogue, ainsi qu'une butée (non représentée) permettent de maintenir le fût 4 dans la position souhaitée.

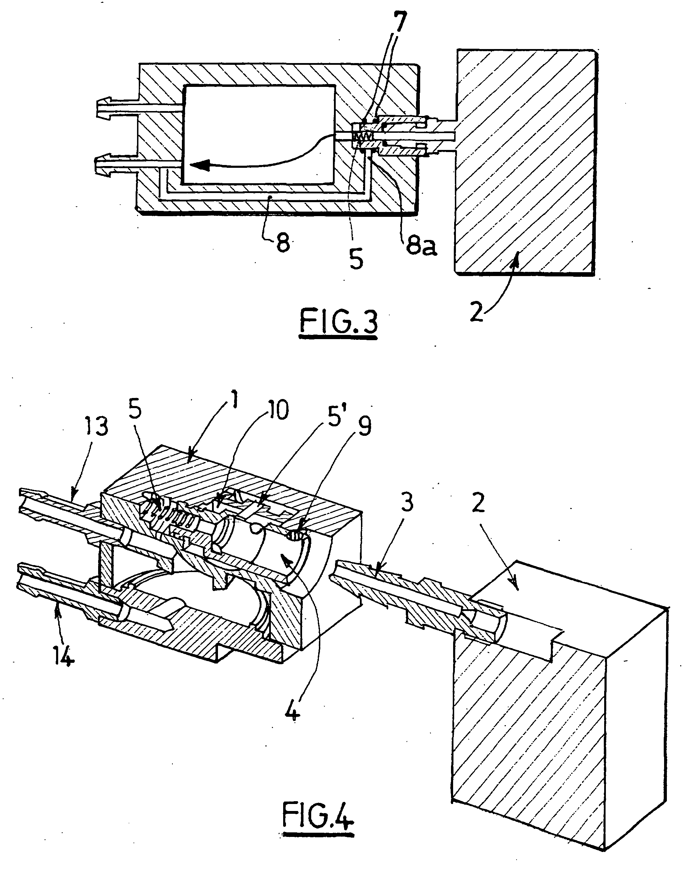

Selon l'invention, la valve 1 est à au moins deux positions stables de raccordement, à savoir une position intermédiaire qui permet le fonctionnement en mode continu de l'ensemble connecté sur le raccord (figure 2) et une position dite "en butée" qui met en oeuvre le mode économiseur de la valve 1 (figure 3), assurant un envoi discontinu du gaz en fonction de la demande en gaz du patient.

Comme montré sur la figure 2, en position intermédiaire, la valve économiseuse se glisse sur l'olive du débitmètre qui se loge dans le fût 4, l'étanchéité entre ces deux pièces étant réalisée par un joint torique 6. De même, l'étanchéité entre le fût 4 et le corps 1 de la économiseuse est réalisée par deux autres joints toriques 7.

Dans cette position, le gaz provenant du débitmètre passe par un canal interne 8 de dérivation qui communique directement avec la sortie dite de « puissance » de la valve, laquelle alimente les voies respiratoires du patient en gaz. La présence de ce passage 8 permet d'éviter le passage du gaz dans le mécanisme de la valve économiseuse, ce qui assure un fonctionnement de l'ensemble en mode à débit continu.

Par ailleurs, comme montré sur la figure 3, si une force de pression supplémentaire est exercée par un opérateur (utilisateur ou personnel médical) sur le corps 1 de la valve économiseuse, dans le sens allant de l'axe de l'olive vers le débitmètre, alors le fût 4 mobile se déplace en translation dans une autre position stable dans laquelle l'entrée du canal 8 se trouve obstruée par les deux joints 7.

Dans cette position dite "en butée", le gaz provenant du débitmètre est contraint de passer par le mécanisme de valve économiseuse et la valve joue alors son rôle de manière classique, c'est-à-dire qu'un débit de gaz sera envoyé vers le patient uniquement lorsqu'une dépression sera créée au niveau de la sortie dite de « détection » du corps 1 de valve, ladite sortie de détection étant reliée pneumatique aux voies respiratoires du patient, par exemple au moyen d'un tuyau flexible de gaz. Dit autrement, le fonctionnement de la valve à la demande est en mode "économie" ou à la demande. Dans tous les cas, la sortie de gaz s'effectue toujours par la sortie dite de « puissance » du corps 1 de valve.

Les figures 4 à 6 sont un exemple de réalisation de la valve de l'invention en vue partielle en trois dimension et en coupe permettant de comprendre le fonctionnement du mécanisme d'accrochage de la valve sur l'olive ou raccord 3 du débitmètre 2.

Comme on peut le voir, le fût 4 de la valve est maintenu sur une butée 5' solidaire d'un bouton 10 par le ressort 5. Dans cette position, les trois billes 9 sont libres dans leur logement respectif dans le fût 4, ces billes 9 servant à assurer l'accrochage de l'olive 3 sur le fût 4, lorsque celle-ci est connectée au raccord 2.

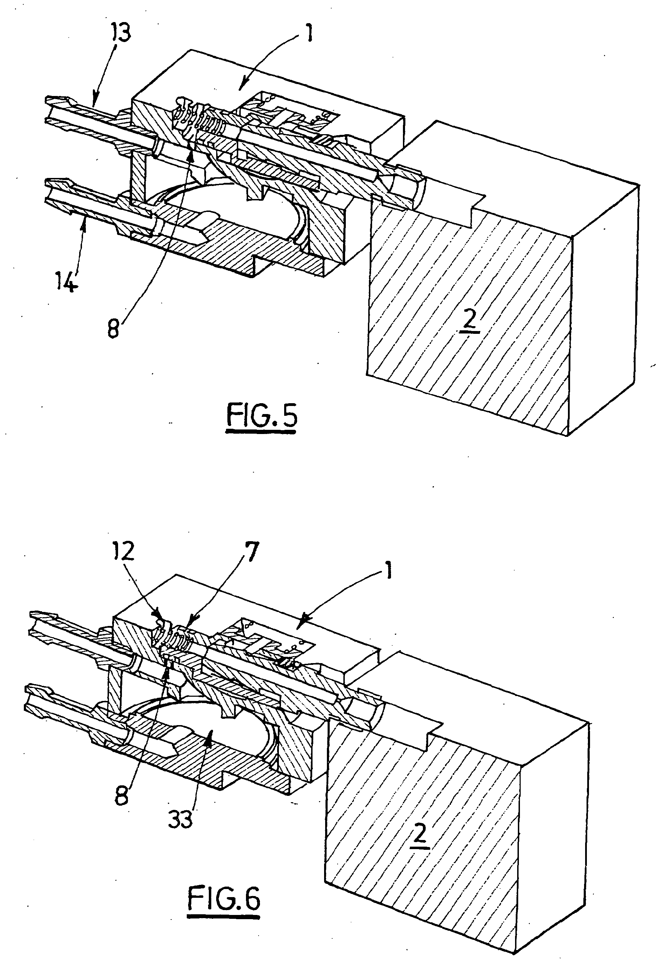

En position intermédiaire (figure 5), le gaz passe par le canal 8 de dérivation puis par la sortie de « puissance. Dans cette position, les trois billes 9 sont contraintes dans la gorge de l'olive 3 du débitmètre 2, ce qui assure l'accrochage de la valve sur celui-ci.

Par ailleurs, lorsque la valve à la demande est en position butée (figure 6), le canal 8 se trouve obstrué par les deux joints toriques 7, le gaz passe alors par le conduit 12 qui communique avec le mécanisme 33 de valve à la demande. La valve à la demande fonctionne alors en mode économiseuse.

La position stable dans chacune des positions, intermédiaire ou butée, est obtenue grâce au positionnement des 3 billes 9 qui coopèrent avec une pente réalisée dans le logement recevant le fût 4. Lorsque le fût prend une position arrière par poussée axiale, ceci a pour effet un rapprochement des billes vers l'axe du fût, celles-ci se logeant alors dans la gorge ménagée dans l'olive 3, la retenant ainsi à l'intérieur du fût. Un verrouillage s'obtient par le rappel du bouton 10 grâce à un ressort axial adjacent placé à son extrémité. A l'inverse, le déverrouillage de l'ensemble s'opère par simple pression digitale sur le bouton 10 permettant le recul du fût et de la libération des billes vers un diamètre extérieur plus grand, ce qui a pour effet de libérer la gorge ménagée dans l'olive 3, celle-ci pouvant ainsi être dégagée du fût mobile 4.

Selon l'invention, la pièce-manchon 4 mobile en forme de fût est donc apte à prendre au moins deux positions stables, à savoir :
- une première position stable dans laquelle le gaz peut traverser, de manière continue, successivement l'évidemment central de la pièce-manchon, le passage secondaire 8 d'acheminement de gaz faisant office de canalisation de dérivation et la portion aval du passage principal d'acheminement de gaz située en aval desdits moyens à valve 33 pour aboutir à l'orifice de sortie de gaz, c'est-à-dire la sortie de puissance, et
- une deuxième position stable dans laquelle le gaz peut traverser successivement l'évidemment central de la pièce-manchon 4, les moyens à valve et la portion aval du passage principal d'acheminement de gaz située en aval desdits moyens à valve pour aboutir à l'orifice de sortie de gaz, et dans laquelle l'entrée amont du passage secondaire 8 d'acheminement de gaz se raccordant fluidiquement au passage principal d'acheminement de gaz en amont desdits moyens à valve est obturée, de préférence l'entrée amont du passage secondaire est obturée par les moyens d'étanchéité, tels des joints toriques, portés par la paroi périphérique externe de la pièce-manchon 4.

Le système à connecteur rapide à deux positions de raccordement à la source de gaz ou débitmètre permet, de par sa position, un fonctionnement de la valve économiseuse en mode continu ou en mode discontinu.

La valve de l'invention ou un ensemble valve/débitmètre est utilisable en oxygénothérapie.

## Revendications

1. Valve de distribution de gaz, en particulier du type valve à la demande, comprenant un corps (1) de valve dans lequel est aménagé:
- un passage principal (11) d'acheminement de gaz comprenant un orifice d'entrée de gaz (12) et un orifice de sortie de gaz (13),
- des moyens à valve (33) agencés sur le passage interne (11) d'acheminement de gaz, entre lesdits orifice d'entrée (12) et orifice de sortie (13) de gaz, permettant de contrôler la circulation du gaz dans ledit passage interne (11) principal d'acheminement de gaz,
- une ligne de détection (14) de dépression pneumatique coopérant avec lesdits moyens à valve (33) pour autoriser, au moins temporairement, la circulation de gaz dans ledit passage interne (11) principal d'acheminement de gaz entre lesdits orifice d'entrée (12) et orifice de sortie (13),
- un passage secondaire (8) d'acheminement de gaz se raccordant fluidiquement au passage principal (11) d'acheminement de gaz en amont (11a) et en aval (11b) desdits moyens à valve (33),
la valve de distribution de gaz comportant des moyens de connexion (4, 9, 5', 10) permettant de raccorder fluidiquement et solidairement ledit corps (1) de valve à un organe d'alimentation (3) en gaz de manière à pouvoir alimenter en gaz ledit passage principal (11) d'acheminement de gaz via ledit orifice (12) d'entrée de gaz,
**caractérisée en ce que** les moyens de connexion (4, 9, 5', 10) comprennent une pièce-manchon (4) mobile en translation dans un logement formant l'entrée (12) du passage (11) principal d'acheminement de gaz, ladite pièce-manchon (4) comportant un évidemment central permettant au gaz de traverser ladite pièce-manchon (4), et la pièce-manchon (4) mobile étant apte à prendre au moins :
. une première position stable de distribution continue de gaz, dans laquelle le gaz est acheminé par le passage secondaire (8) d'acheminement de gaz pour être délivré de manière continue par l'orifice de sortie (13) de gaz, et
. une deuxième position stable de distribution discontinue de gaz, dans laquelle le gaz traverse les moyens à valve (33) pour être délivré de manière discontinue par l'orifice de sortie de gaz (13) et uniquement lorsque une dépression apparaît dans la ligne de détection (14) de dépression.

2. Valve selon la revendication 1, **caractérisée en ce qu'**un logement est aménagé sur le passage principal (11) d'acheminement de gaz, une paroi dudit logement portant l'orifice d'entrée (12) de gaz et au moins une partie des moyens de connexion (4, 9, 5', 10) étant situés dans ledit logement ou au niveau de la paroi dudit logement.

3. Valve selon l'une des revendications 1 ou 2, **caractérisée en ce que** la pièce-manchon (4) porte des moyens d'étanchéité (7) sur sa paroi périphérique externe.

4. Valve selon la revendication 3, **caractérisée en ce que** les moyens d'étanchéité (7) sont deux joints toriques espacés l'un de l'autre.

5. Valve selon l'une des revendications 3 à 4, **caractérisée en ce que**, lorsque la pièce-manchon (4) est dans sa deuxième position stable, les moyens d'étanchéité (7) portés par la paroi périphérique externe de la pièce-manchon (4) empêchent toute introduction de gaz dans l'entrée (8a) amont du passage secondaire (8) d'acheminement de gaz se raccordant fluidiquement au passage principal (11) d'acheminement de gaz en amont desdits moyens à valve (33).

6. Valve selon l'une des revendications 1 à 5, **caractérisée en ce qu'**un mécanisme de maintien permet un maintien de la pièce-manchon (4) dans sa première position stable et/ou dans sa deuxième position stable.

7. Valve selon la revendication 6, **caractérisée en ce qu'**un mécanisme de libération coopérant avec le mécanisme de maintien permet la libération de la pièce-manchon (4) lorsqu'elle est dans la première position stable et/ou dans la deuxième position stable.

8. Valve selon la revendication 7, **caractérisée en ce que** le mécanisme de libération comprend un bouton (10) à actionnement digital et d'un ressort de rappel lui permettant de reprendre sa position lorsque celui-ci n'est pas actionné.

9. Valve selon la revendication 7, **caractérisée en ce que** le mécanisme de libération comprend un bouton (10) à actionnement digital.

10. Valve selon l'une des revendications 1 à 9, **caractérisée en ce qu'**un moyen élastique (5) situé dans le passage principal (11) d'acheminement de gaz exerce une force de poussée sur la pièce-manchon (4) dans le sens tendant à l'éloigner de l'entrée (8a) amont du passage secondaire (8).

11. Ensemble formé par un débitmètre (2) comportant un raccord (3) de sortie sur lequel est connecté de manière solidaire une valve selon l'une des revendications 1 à 10.

## Claims

1. Gas delivery valve, in particular of the demand-valve type, having a valve body (1) in which is made:
- a main passage (11) for routing gas comprising a gas inlet orifice (12) and a gas outlet orifice (13),
- valve means (33) arranged on the internal passage (11) for routing gas, between the said gas inlet orifice (12) and outlet orifice (13), making it possible to control the flow of gas in the said main internal passage (11) for routing gas,
- a line (14) for detecting a pneumatic vacuum cooperating with the said valve means (33) in order to allow, at least temporarily, the flow of gas in the said main internal passage (11) for routing gas between the said inlet orifice (12) and outlet orifice (13),
- a secondary passage (8) for routing gas which is fluidically connected to the main gas routing passage (11) upstream (11a) and downstream (11b) of the said valve means (33),
the gas delivery valve comprising connection means (4, 9, 5', 10) making it possible to connect the said valve body (1) fluidically and securely to a gas feed member (3) so that the said main gas routing passage (11) can be fed with gas via the said gas inlet orifice (12), **characterized in that** the connection means (4, 9, 5' 10) comprise a sleeve piece (4) which can be moved in translation in a housing forming the inlet (12) of the main gas routing passage (11), the said sleeve piece (4) having a central recess making it possible for the gas to pass through the said sleeve piece (4), and the moveable sleeve piece (4) being capable of taking at least:
- a first stable position for continuous gas delivery, in which the gas is routed via the secondary gas routing passage (8) in order to be delivered continuously via the gas outlet orifice (13), and
- a second stable position for discontinuous gas delivery, in which the gas passes through the valve means (33) so as to be delivered discontinuously via the gas outlet orifice (13) and only when a vacuum appears in the vacuum detection line (14).

2. Valve according to Claim 1, **characterized in that** a housing is made on the main gas routing passage (11), one wall of the said housing bearing the gas inlet orifice (12) and at least part of the connection means (4, 9, 5', 10) being located in the said housing or in the wall of the said housing.

3. Valve according to either of Claims 1 and 2, **characterized in that** the sleeve piece (4) bears sealing means (7) on its outer peripheral wall.

4. Valve according to Claim 3, **characterized in that** the sealing means (7) are two O-ring seals spaced apart from each other.

5. Valve according to one of Claims 3 to 4, **characterized in that**, when the sleeve piece (4) is in its second stable position, the sealing means (7) borne by the outer peripheral wall of the sleeve piece (4) prevent any introduction of gas into the upstream inlet (8a) of the secondary gas routing passage (8) which is fluidically connected to the main gas routing passage (11) upstream of the said valve means (33).

6. valve according to one of Claims 1 to 5, **characterized in that** a holding mechanism allows the sleeve piece (4) to be held in its first stable position and/or in its second stable position.

7. Valve according to Claim 6, **characterized in that** a release mechanism cooperating with the holding mechanism allows the sleeve piece (4) to be released when it is in the first stable position and/or in the second stable position.

8. Valve according to Claim 7, **characterized in that** the release mechanism comprises a finger-actuated button (10) and a return spring allowing it to return to its position when it is not actuated.

9. Valve according to Claim 7, **characterized in that** the release mechanism has a finger-actuated button (10) .

10. Valve according to one of Claims 1 to 9, **characterized in that** an elastic means (5) located in the main gas routing passage (11) exerts a thrust force on the sleeve piece (4) in the direction tending to move away from the upstream inlet (8a) of the secondary passage (8).

11. Unit formed by a flowmeter (2) comprising an outlet connection (3) to which a valve according to one of Claims 1 to 10 is securely connected

## Patentansprüche

1. Gasversorgungsventil, insbesondere von der Art bedarfsmäßig arbeitendes Ventil, das einen Ventilkörper (1) aufweist, in dem ausgebildet sind:
- ein Hauptgasbeförderungsdurchlass (11), der eine Gaseinlassöffnung (12) und eine Gasauslassöffnung (13) aufweist,
- Ventileinrichtungen (33), die auf dem inneren Gasbeförderungsdurchlass (11) zwischen der Gaseinlassöffnung (12) und der Gasauslassöffnung (13) angeordnet sind und es ermöglichen, den Gaskreislauf im inneren Hauptgasbeförderungsdurchlass (11) zu steuern,
- eine Leitung (14) zur Erfassung eines pneumatischen Unterdrucks, die mit den Ventileinrichtungen (33) zusammenwirkt, um zumindest zeitweise den Gaskreislauf im inneren Hauptgasbeförderungsdurchlass (11) zwischen der Einlassöffnung (12) und der Auslassöffnung (13) zu erlauben,
- ein Nebengasbeförderungsdurchlass (8), der vor (11a) und hinter (11b) den Ventileinrichtungen (33) fluidisch an den Hauptgasbeförderungsdurchlass (11) angeschlossen ist,
wobei das Gasversorgungsventil Verbindungseinrichtungen (4, 9, 5', 10) aufweist, die es ermöglichen, den Ventilkörper (1) fluidisch und fest mit einem Gaszufuhrelement (3) zu verbinden, um den Hauptgasbeförderungsdurchlass (11) über die Gaseinlassöffnung (12) mit Gas speisen zu können,
**dadurch gekennzeichnet, dass** die Verbindungseinrichtungen (4, 9, 5', 10) ein Muffenteil (4) aufweisen, das in einem den Einlass (12) des Hauptgasbeförderungsdurchlasses (11) bildenden Sitz translationsbeweglich ist, wobei das Muffenteil (4) eine zentrale Aussparung aufweist, die es dem Gas ermöglicht, das Muffenteil (4) zu durchqueren, und das bewegliche Muffenteil (4) in der Lage ist, mindestens:
. eine erste stabile Stellung der kontinuierlichen Gasversorgung einzunehmen, in der das Gas vom Nebengasbeförderungsdurchlass (8) befördert wird, um kontinuierlich durch die Gasauslassöffnung (13) geliefert zu werden, und
. eine zweite stabile Stellung der diskontinuierlichen Gasversorgung einzunehmen, in der das Gas die Ventileinrichtungen (33) durchquert, um von der Gasauslassöffnung (13) diskontinuierlich und nur dann geliefert zu werden, wenn ein Unterdruck in der Unterdruck-Erfassungsleitung (14) auftritt.

2. Ventil nach Anspruch 1, **dadurch gekennzeichnet, dass** ein Sitz auf dem Hauptgasbeförderungsdurchlass (11) ausgebildet ist, wobei eine Wand des Sitzes die Gaseinlassöffnung (12) trägt und mindestens ein Teil der Verbindungseinrichtungen (4, 9, 5', 10) sich in dem Sitz oder in Höhe der Wand des Sitzes befindet.

3. Ventil nach einem der Ansprüche 1 oder 2, **dadurch gekennzeichnet, dass** das Muffenteil (4) auf seiner äußeren Umfangswand Dichtungsmittel (7) trägt.

4. Ventil nach Anspruch 3, **dadurch gekennzeichnet, dass** die Dichtungsmittel (7) zwei Ringdichtungen sind, die in Abstand zueinander angeordnet sind.

5. Ventil nach einem der Ansprüche 3 bis 4, **dadurch gekennzeichnet, dass**, wenn das Muffenteil (4) in seiner zweiten stabilen Stellung ist, die von der äußeren Umfangswand des Muffenteils (4) getragenen Dichtungsmittel (7) jede Einführung von Gas in den vorderen Einlass (8a) des Nebengasbeförderungsdurchlasses (8) verhindern, der vor den Ventileinrichtungen (33) fluidisch mit dem Hauptgasbeförderungsdurchlass (11) verbunden ist.

6. Ventil nach einem der Ansprüche 1 bis 5, **dadurch gekennzeichnet, dass** ein Haltemechanismus einen Halt des Muffenteils (4) in seiner ersten stabilen Stellung und/oder in seiner zweiten stabilen Stellung ermöglicht.

7. Ventil nach Anspruch 6, **dadurch gekennzeichnet, dass** ein Freigabemechanismus, der mit dem Haltemechanismus zusammenwirkt, die Freigabe des Muffenteils (4) ermöglicht, wenn es in der ersten stabilen Stellung und/oder in der zweiten stabilen Stellung ist.

8. Ventil nach Anspruch 7, **dadurch gekennzeichnet, dass** der Freigabemechanismus einen Knopf (10) mit digitaler Betätigung und eine Rückstellfeder aufweist, die es ihm ermöglicht, seine Stellung wieder einzunehmen, wenn er nicht betätigt wird.

9. Ventil nach Anspruch 7, **dadurch gekennzeichnet, dass** der Freigabemechanismus einen Knopf (10) mit digitaler Betätigung aufweist.

10. Ventil nach einem der Ansprüche 1 bis 9, **dadurch gekennzeichnet, dass** ein elastisches Mittel (5), das sich im Hauptgasbeförderungsdurchlass (11) befindet, auf das Muffenteil (4) eine Schubkraft in der Richtung ausübt, die es von dem vorderen Einlass (8a) des Nebendurchlasses (8) entfernt.

11. Einheit, die von einem Durchflussmesser (2) gebildet wird, der einen Ausgangsanschluss (3) aufweist, mit dem ein Ventil nach einem der Ansprüche 1 bis 10 fest verbunden ist.
